# EUROPÄISCHE PATENTSCHRIFT

(11) **EP 2 086 500 B1**
(45) Veröffentlichungstag und Bekanntmachung des Hinweises auf die Patenterteilung: **28.07.2010**
(21) Anmeldenummer: 07818320.9
(22) Anmeldetag: 21.09.2007
(51) Int. Cl.: A61K 8/49, A61K 8/35, A61K 8/44, A61K 8/37, A61Q 17/04

(54) **KOSMETISCHE ODER DERMATOLOGISCHE ZUBEREITUNGEN MIT KOMBINATIONEN AUS (R)-2-[((1R, 2S, 5R)-2-ISOPROPYL-5-METHYL-CYCLOHEXANECARBONYL)-AMINO]-PROPIONSÄURE METHYLESTER UND EINER ODER MEHREREN LICHTSCHUTZFILTERSUBSTANZEN**
COSMETIC OR DERMATOLOGICAL PREPARATIONS WITH COMBINATIONS OF METHYL (R)-2-[((1R, 2S, 5R)-2-ISOPROPYL-5-METHYL-CYCLOHEXANECARBONYL)-AMINO]-PROPIONATE AND ONE OR MORE LIGHT PROTECTIVE FILTER SUBSTANCES
PRÉPARATIONS COSMÉTIQUES OU DERMATOLOGIQUES COMPRENANT DES COMBINAISONS D'ESTER MÉTHYLIQUE DE L'ACIDE (R)-2-[((1R, 2S, 5R)-2-ISOPROPYL-5-METHYL-CYCLOHEXANECARBONYL)-AMINO]-PROPIONIQUE ET D'UNE OU DE PLUSIEURS SUBSTANCES DE FILTRE UV

(30) Priorität: 29.09.2006 DE 102006047162
(43) Veröffentlichungstag der Anmeldung: 12.08.2009
(73) Patentinhaber: Beiersdorf AG, 20253 Hamburg (DE)
(72) Erfinder: ECKERT, Julia, 22085 Hamburg (DE); KOLBE, Ludger, 21255 Dohren (DE); NEUFANG, Gitta, 20149 Hamburg (DE)
(74) Vertreter: Hartmann, Jost
(86) Internationale Anmeldenummer: PCT/EP2007/008230
(87) Internationale Veröffentlichungsnummer: WO 2008/040457

(56) Entgegenhaltungen:
- EP-A- 1 639 993
- DE-A1- 2 205 255
- WATSON H R ET AL: "NEW COMPOUNDS WITH THE MENTHOL COOLING EFFECT" JOURNAL OF THE SOCIETY COSMETIC CHEMISTS, SOCIETY OF COSMETIC CHEMISTS,, US, Bd. 29, Nr. 4, 1978, Seiten 185-200, XP009045124 ISSN: 0037-9832

## Beschreibung

Die vorliegende Erfindung betrifft kosmetische oder dermatologische Zubereitungen mit Kombinationen aus (R)-2-[((1R,2S,5R)-2-isopropyl-5-methyl-cyclohexanecarbonyl)-amino]-propionsäuremethylester (synonym: 2-Isopropyl-5-Methyl-Cyclohexancarbonyl-D-Alaninmethylester) und einer oder mehreren Lichtschutzfiltersubstanzen sowie kosmetische und dermatologische Lichtschutzzubereitungen mit einem Gehalt an solchen Kombinationen.

Die schädigende Wirkung des ultravioletten Teils der Sonnenstrahlung auf die Haut ist allgemein bekannt. Während Strahlen mit einer Wellenlänge, die kleiner als 290 nm ist (der so genannte UVC-Bereich), von der Ozonschicht in der Erdatmosphäre absorbiert werden, verursachen Strahlen im Bereich zwischen 290 nm und 320 nm, dem so genannten UVB-Bereich, ein Erythem, einen einfachen Sonnenbrand oder sogar mehr oder weniger starke Verbrennungen.

Als ein Maximum der Erythemwirksamkeit des Sonnenlichtes wird der engere Bereich um 308 nm angegeben.

Zum Schutze gegen UVB-Strahlung sind zahlreiche Verbindungen bekannt, bei denen es sich zumeist um Derivate des 3-Benzylidencamphers, der 4-Aminobenzoësäure, der Zimtsäure, der Salicylsäure, des Benzophenons sowie auch des 2-Phenylbenzimidazols handelt.

Auch für den Bereich zwischen etwa 320 nm und etwa 400 nm, den sogenannten UVA-Bereich, ist es wichtig, Filtersubstanzen zur Verfügung zu haben, da auch dessen Strahlen Schäden hervorrufen können. So ist erwiesen, dass UVA-Strahlung zu einer Schädigung der elastischen und kollagenen Fasern des Bindegewebes führt, was die Haut vorzeitig altern läßt, und dass sie als Ursache zahlreicher phototoxischer und photoallergischer Reaktionen zu sehen ist. Der schädigende Einfluß der UVB-Strahlung kann durch UVA-Strahlung verstärkt werden.

UV-A-Strahlung kann ferner Hautschädigungen hervorrufen, indem das hauteigene Keratin oder Elastin geschädigt wird. Hierdurch werden Elastizität und Wasserspeichervermögen der Haut reduziert, d.h. die Haut wird weniger geschmeidig und neigt zur Faltenbildung. Die auffallend hohe Hautkrebshäufigkeit in Gegenden starker Sonneneinstrahlung zeiqt, dass offenbar auch Schädigungen der Erbinformationen in den Zellen durch Sonnenlicht, speziell durch UV-A-Strahlung, hervorgerufen werden.

Die UV-Strahlung kann aber auch zu photochemischen Reaktionen führen, wobei dann die photochemischen Reaktionsprodukte in den Hautmetabolismus eingreifen.

Vorwiegend handelt es sich bei solchen photochemischen Reaktionsprodukten um radikalische Verbindungen, z.B. Hydroxylradikale. Auch undefinierte radikalische Photoprodukte, welche in der Haut selbst entstehen, können aufgrund ihrer hohen Reaktivität unkontrollierte Folgereaktionen an den Tag legen. Aber auch Singulettsauerstoff, ein nichtradikalischer angeregter Zustand des Sauerstoffmoleküls kann bei UV-Bestrahlung auftreten, ebenso kurzlebige Epoxide und viele Andere. Singulettsauerstoff beispielsweise zeichnet sich gegenüber dem normalerweise vorliegenden Triplettsauerstoff (radikalischer Grundzustand) durch gesteigerte Reaktivität aus. Allerdings existieren auch angeregte, reaktive (radikalische) Triplettzustände des Sauerstoffmoleküls.

Ferner zählt UV-Strahlung zur ionisierenden Strahlung. Es besteht also das Risiko, dass auch ionische Spezies bei UV-Exposition entstehen, welche dann ihrerseits oxidativ in die biochemischen Prozesse einzugreifen vermögen.

Die Pigmentierung der menschlichen Haut, wird im wesentlichen durch die Gegenwart von Melanin bewirkt. Melanin und dessen Abbauprodukte (Melanoide), Carotin, Durchblutungsgrad sowie die Beschaffenheit und Dicke des Stratum corneum und andere Hautschichten lassen Hautfarbtöne von praktisch weiß (bei verringerter Füllung oder bei Fehlen der Blutgefäße) oder gelblich über hellbraun-rötlich, bläulich bis braun verschiedener Nuancen und schließlich beinahe schwarz erscheinen. Die einzelnen Hautregionen zeigen aufgrund unterschiedlicher Melanin-Mengen unterschiedliche Tiefe der Farbtönung.

Das natürliche Melanin schützt die Haut vor eindringender UV-Strahlung. Die Anzahl der in den Melanocyten produzierten Melanin-Granula entscheidet über Hell-od. Dunkelhäutigkeit. Bei starker Pigmentierung (z.B. bei Farbigen, aber auch bei Hellhäutigen nach einiger UV-Bestrahlung) ist Melanin auch im Stratum spinosum und sogar im Stratum corneum festzustellen. Es schwächt die UV-Strahlung um bis zu ca. 90%, bevor diese das Corium erreicht.

Je nach Lichtempfindlichkeit werden in der Regel folgende Hauttypen unterschieden:
Hauttyp I bräunt nie, bekommt immer einen Sonnenbrand.
Hauttyp II bräunt kaum, bekommt leicht einen Sonnenbrand.
Hauttyp III bräunt durchschnittlich gut.
Hauttyp IV bräunt leicht und anhaltend, bekommt fast nie Sonnenbrand.
Hauttyp V dunkle, oft fast schwarze Haut, bekommt nie Sonnenbrand.

Die natürliche Abschirmung der schädlichen UV-Strahlung ist ein handfester Vorteil der natürlichen Hautbräunung. Seit einigen Jahrzehnten gilt darüberhinaus eine "gesunde" Hautfarbe als Zeichen von insbesondere sportlicher Aktivität und wird daher von einer breiten Verbraucherschicht als erstrebenswert erachtet. Vertreter der Hauttypen I und II, die sich einer solchen Hauttönung erfreuen wollen, sind daher ohnehin auf selbstbräunende Präparate angewiesen. Aber auch Vertreter des Hauttyps III, die sich nicht allzusehr den Risiken des Sonnenbades aussetzen und trotzdem gebräunt aussehen wollen, sind dankbare Zielgruppen für selbstbräunende Zubereitungen.

Ein indirekter Nachteil von Lichtschutzzubereitungen besteht darin, dass sie unter Bedingungen angewandt werden (verstärkte Sonneneinstrahlung), unter welchen die Haut verstärkt austrocknet. Wünschenswert ist, Lichtschutzzubereitungen zur Verfügung zu stellen, die der Hautaustrocknung entgegenwirken. Auch wäre im allgemeinen bei der erhöhten Temperatur, die mit intensiverer Sonneneinstrahlung verbunden ist, erwünscht, dass von der Zubereitung eine gewisse Kühlwirkung ausgeht.

Die Kühlwirkung kosmetischer Zubereitungen beruht bisher auf zwei Grundprinzipien:
Einsatz von Komponenten die sich nach topischer Applikation gasförmig verflüchtigen und die hierfür erforderlich Energiemenge, die sog. Verdampfungsenthalpie, zu einem Großteil der Hautoberfläche entnehmen. Es werden daher in entsprechenden nicht okklusiven Kosmetika geeignete flüssige Komponenten eingesetzt. Als besonders geeignet hat sich hier Ethanol erwiesen, daneben zeigen Formulierungen mit hohem Wassergehalt ebenfalls eine deutliche Kühlwirkung.

Einsatz von sogenannten "Cooling Agents", die mit den Wärmerezeptoren der Haut in Wechselwirkung treten und somit ein Kälteempfinden auslösen, ohne eine meßbare physikalische Abkühlung zu generieren. Hierfür kommen insbesondere Menthol und diverse Mentholderivate (Frescolate, Physcool, Questice L, etc.) zum Einsatz. Insbesondere hohe Ethanolgehalte führen zu einem verstärktem Austrocknen der Haut. Menthol und seine Derivate sind, neben dem irritativen schleimhautreizenden Potential, insbesondere aufgrund ihres deutlichen Eigengeruchs für zahlreiche Einsatzzwecke unter olfaktorischen Gesichtspunkten nicht geeignet. Häufig genug bewirken solche Substanzen darüberhinaus gleichzeitig eine Durchblutungssteigerung, die im Gegenteil ein Wärmegefühl hervorruft.

EP-A-1 639 993 offenbart Shampoo oder Körperreinigungszubereitungen, die neben einem kühlenden Stoff Sonnenschutzmitteln enthalten können. Die als kühlende Substanzen einsetzbaren N-substituierten Carboxamide sind mit C1-10 Alkyl- oder Alkenylgruppen substituiert (R= C1-10 Alkyl, Alkenyl).

DE-A-22 05 255 offenbart N-substituierte Carboxamide als kühlende Substanzen in kosmetischen Zubereitungen mit R= -CH(CH₃)COOC₂H₅, -CH₂CH₂COOC₂H₅, - CH₂COOCH₃, -CH(CH₃)CH₂COOC₂H₅.

H. Watson et al.: "New Compounds with the Menthol Cooling Effect" Journal of the Society of Cosmetic Chemists, Bd. 29, Nr. 4, 1978, Seiten 185-200, offenbart als N-substituiertes Carboxamid mit mentholartigem Kühleffekt Isopropyl-5-Methyl-Cyclohexancarbonyl-Alaninethylester (R= -CH(CH₃)COOC₂H₅).

Den Nachteilen des Standes der Technik abzuhelfen, war also Aufgabe der vorliegenden Erfindung.

Es war indes überraschend und für den Fachmann nicht vorauszusehen, dass kosmetische oder dermatologische Zubereitungen mit einem Gehalt Wirkstoffkombinationen aus 2-Isopropyl-5-Methyl-Cyclohexancarbonyl-D-Alaninmethylester und einer oder mehreren Lichtschutzfiltersubstanzen den Nachteilen des Standes der Technik abhelfen.

Bei Befolgen der erfindungsgemäßen Lehre sind Zubereitungen erhältlich, welche hervorragenden UV-Schutz bewirken und die Haut trotzdem kühlen und gut befeuchten, ohne dass die Produkte einen Mentholgeruch aufweisen.

2-lsopropyl-5-Methyl-Cyclohexancarbonyl-D-Alaninmethylester zeichnet sich durch folgende Struktur aus: 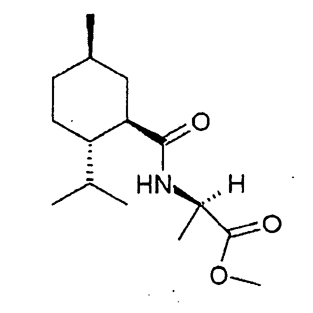 Die Synthese von 2-Isopropyl-5-Methyl-Cyclohexancarbonyl-D-Alaninmethylester [synonym: (R)-2-[((1R,2S,5R)-2-isopropyl-5-methyl-cyclohexanecarbonyl)-amino]-propionsäuremethylester kann nach folgender Vorschrift erfolgen:
1,0 g D-Alaninmethylesterhydrochlorid (von Aldrich Chemical Co erhalten) wurde in 28 ml diethylether und 1 ml doppeltdestillierten Wassers aufgelöst und auf 0 °C gekühlt. Eine Spatelspitze des Katalysators Diaminopyrimidin wurde hinzugefügt. 1,62 ml p-Menthylchlorid wurden tropfenweise hinzugefügt, gefolgt von 2 ml Triethylamin. Flocken eines falrblosen Niederschlages bildeten sich in der Mischung, die über Nacht bei Raumtemperatur gerührt wurde. Der Niederschlag wurde in Ethylacetat gelöst, mit doppelt destilliertem Wasser gewaschen und über Natriumsulfat getrocknet. Die organische Phase wurde unter vermindertem Druck verdampft, wobei 2 g Endprodukt erhalten wurde, welches bei Raumtemperatur kristallisierte. Die erwartete Molekularmasse und Struktur wurden mit Massenspektrometer bzw. anhand des NMR-Spektrums bestätigt.

Erfindungsgemäße Zubereitungen sind vorteilhaft, welche dadurch gekennzeichnet sind, dass sie 0,001 Gew.-% bis 10 Gew.-%, bevorzugt 0,01 Gew.-% bis 1,0 Gew.-%, insbesondere 0,05 - 0,5 Gew.-%, bezogen auf das Gesamtgewicht der Zubereitungen, an 2-Isopropyl-5-Methyl-Cyclohexancarbonyl-D-Alaninmethylester enthalten. Erfindungsgemäß vorteilhafte Lichtschutzfilter sind Substanzen, die UV-Strahlung im UV-A-und/oder UV-B-Bereich absorbieren, wobei die Gesamtmenge der Filtersubstanzen z. B. 0,1Gew.-% bis 30 Gew.-%, vorzugsweise 0,5 bis 20 Gew.-%, insbesondere 1,0 bis 15,0 Gew.-% beträgt, bezogen auf das Gesamtgewicht der Zubereitungen, um kosmetische Zubereitungen zur Verfügung zu stellen, die das Haar bzw. die Haut vor dem gesamten Bereich der ultravioletten Strahlung schützen. Sie können auch als Sonnenschutzmittel fürs Haar oder die Haut dienen.

Vorteilhafte UV-A-Filtersubstanzen im Sinne der vorliegenden Erfindung sind Dibenzoylmethanderivate, insbesondere das 4-(tert.-Butyl)-4'-methoxydibenzoylmethan (CAS-Nr. 70356-09-1), welches von Givaudan unter der Marke Parsol® 1789 und von Merck unter der Handelsbezeichnung Eusolex® 9020 verkauft wird.

Weitere vorteilhafte UV-A-Filtersubstanzen sind die Phenylen-1,4-bis-(2-benzimidazyl)-3,3'-5,5'-tetrasulfonsäure 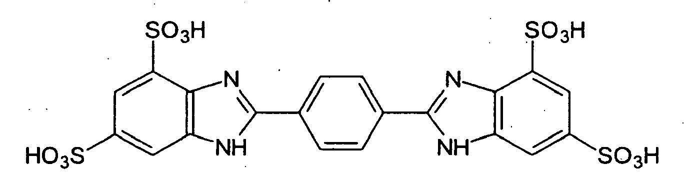 und ihre Salze, besonders die entsprechenden Natrium-, Kalium- oder Triethanolammonium-Salze, insbesondere das Phenylen-1,4-bis-(2-benzimidazyl)-3,3'-5,5'-tetrasulfonsäure-bis-natriumsalz 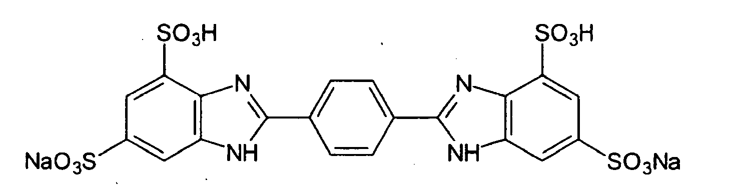 mit der INCI-Bezeichnung Bisimidazylate, welches beispielsweise unter der Handelsbezeichnung Neo Heliopan AP bei Haarmann & Reimer erhältlich ist.

Ferner vorteilhaft sind das 1,4-di(2-oxo-10-Sulfo-3-bornylidenmethyl)-Benzol und dessen Salze (besonders die entprechenden 10-Sulfato-verbindungen, insbesondere das entsprechende Natrium-, Kalium- oder Triethanolammonium-Salz), das auch als Benzol-1,4-di(2-oxo-3-bofnylidenmethyl-10-sulfonsäure) bezeichnet wird und sich durch die folgende Struktur auszeichnet:

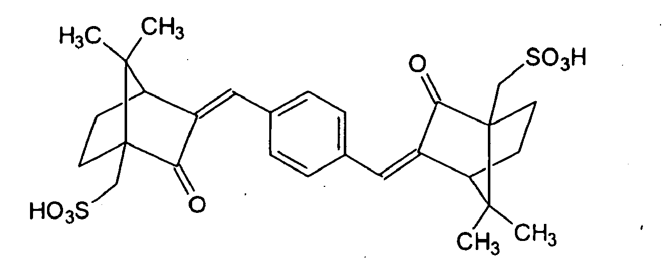

Vorteilhafte UV-Filtersubstanzen im Sinne der vorliegenden Erfindung sind ferner sogenannte Breitbandfilter, d.h. Filtersubstanzen, die sowohl UV-A- als auch UV-B-Strahlung absorbieren.

Vorteilhafte Breitbandfilter oder UV-B-Filtersubstanzen sind beispielsweise Bis-Resorcinyltriazinderivate mit der folgenden Struktur: 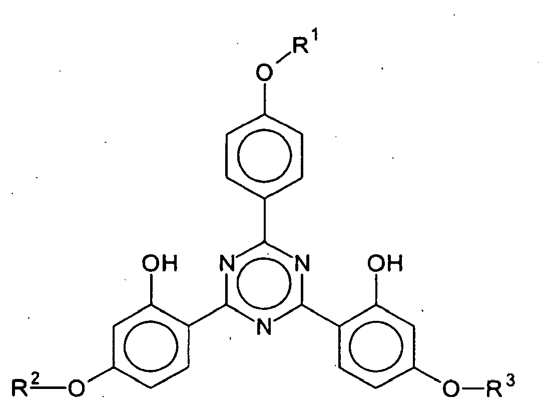 wobei R1, R2 und R3 unabhängig voneinander gewählt werden aus der Gruppe der verzweigten und unverzweigten Alkylgruppen mit 1 bis 10 Kohlenstoffatomen bzw. ein einzelnes Wasserstoffatom darstellen. Insbesondere bevorzugt sind das 2,4-Bis-{[4-(2-Ethyl-hexyloxy)-2-hydroxy]-phenyl}-6-(4-methoxyphenyl)-1,3,5-triazin (INCI: Aniso Triazin), welches unter der Handelsbezeichnung Tinosorb® S bei der CIBA-Chemikalien GmbH erhältlich ist, und das 4,4',4"-(1,3,5-Triazin-2,4,6-triyltriimino)-tris-benzoësäure-tris(2-ethylhexylester), synonym: 2,4,6-Tris-[anilino-(p-carbo-2'-ethyl-1'-hexyloxy)]-1,3,5-triazin (INCI: Octyl Triazone), welches von der BASF Aktiengesellschaft unter der Warenbezeichnung UVINUL® T 150 vertrieben wird.

Auch andere UV-Filtersubstanzen, welche das Strukturmotiv 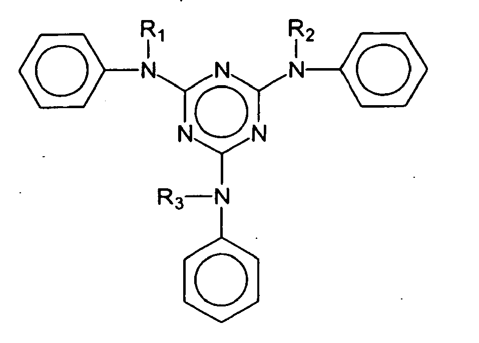 aufweisen, sind vorteilhafte UV-Filtersubstanzen im Sinne der vorliegenden Erfindung, beispielsweise die in der Europäischen Offenlegungsschrift EP 570 838 A1 beschriebenen s-Triazinderivate, deren chemische Struktur durch die generische Formel 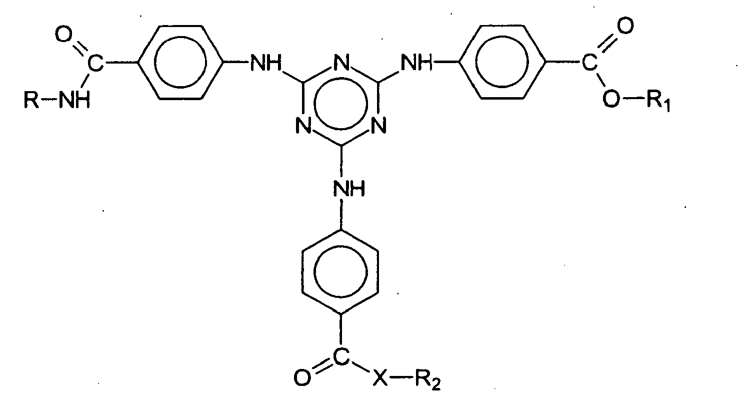 wiedergegeben wird, wobei
R einen verzweigten oder unverzweigten C1-C18-Alkylrest, einen C5-C12-Cycloalkylrest, gegebenenfalls substituiert mit einer oder mehreren C1-C4- Alkylgruppen, darstellt,
X ein Sauerstoffatom oder eine NH-Gruppe darstellt,
R1 einen verzweigten oder unverzweigten C1-C18-Alkylrest, einen C5-C12-Cycloalkylrest, gegebenenfalls substituiert mit einer oder mehreren C1-C4- Alkylgruppen, oder ein Wasserstoffatom, ein Alkalimetallatom, eine Ammoniumgruppe oder eine Gruppe der Formel 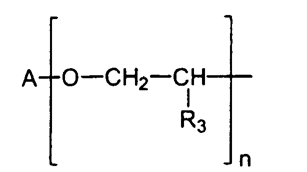 bedeutet, in welcher
- A: einen verzweigten oder unverzweigten C1-C18-Alkylrest, einen C5-C12-Cycloalkyl- oder Arylrest darstellt, gegebenenfalls substituiert mit einer oder mehreren C1-C4- Alkylgruppen,
- R3: ein Wasserstoffatom oder eine Methylgruppe darstellt,
- n: eine Zahl von 1 bis 10 darstellt,
- R2: einen verzweigten oder unverzweigten C1-C18-Alkylrest, einen C5-C12-Cycloalkylrest, gegebenenfalls substituiert mit einer oder mehreren C1-C4- Alkylgruppen, darstellt, wenn X die NH-Gruppe darstellt, und
einen verzweigten oder unverzweigten C1-C18-Alkylrest, einen C5-C12-Cycloalkylrest, gegebenenfalls substituiert mit einer oder mehreren C1-C4- Alkylgruppen, oder ein Wasserstoffatom, ein Alkalimetallatom, eine Ammoniumgruppe oder eine Gruppe der Formel 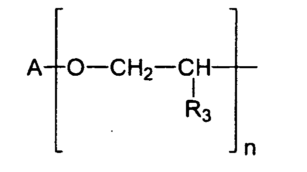
bedeutet, in welcher
- A: einen verzweigten oder unverzweigten C1-C18-Alkylrest, einen C5-C12-Cycloalkyl- oder Arylrest darstellt, gegebenenfalls substituiert mit einer oder mehreren C1-C4-Alkylgruppen,
- R3: ein Wasserstoffatom oder eine Methylgruppe darstellt,
- n: eine Zahl von 1 bis 10 darstellt,
wenn X ein Sauerstoffatom darstellt.

Besonders bevorzugte UV-Filtersubstanz im Sinne der vorliegenden Erfindung ist ferner ein unsymmetrisch substituiertes s-Triazin, dessen chemische Struktur durch die Formel 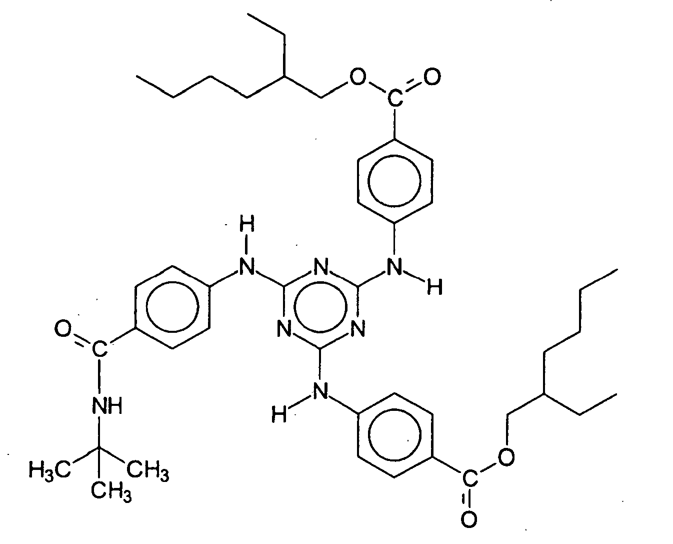 wiedergegeben wird, welches im Folgenden auch als Dioctylbutylamidotriazon (INCI: Dioctylbutamidotriazone) bezeichnet wird und unter der Handelsbezeichnung UVASORB HEB bei Sigma 3V erhältlich ist.

Auch in der Europäischen Offenlegungsschrift 775 698 werden bevorzugt einzusetzende Bis-Resorcinyltriazinderivate beschrieben, deren chemische Struktur durch die generische Formel 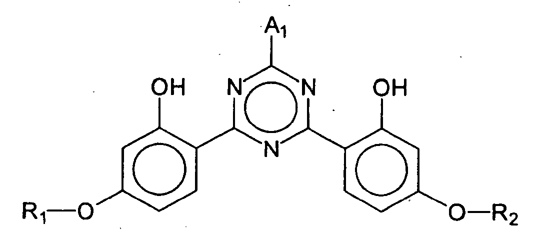 wiedergegeben wird, wobei R1 , R2 und A1 verschiedenste organische Reste repräsentieren.

Vorteilhaft im Sinne der vorliegenden Erfindung sind ferner das 2,4-Bis-{[4-(3-sulfonato)-2-hydroxy-propyloxy)-2-hydroxy]-phenyl}-6-(4-methoxyphenyl)-1,3,5-triazin Natriumsalz, das 2,4-Bis-{[4-(3-(2-Propyloxy)-2-hydroxy-propyloxy)-2-hydroxy]-phenyl}-6-(4-methoxyphenyl)-1,3,5-triazin, das 2,4-Bis-{[4-(2-ethyl-hexyloxy)-hydroxy]-phenyl}-6-[4-(2-methoxyethylcarboxyl)-phenylamino]-1,3,5-triazin, das 2,4-Bis-{[4-(3-(2-propyloxy)-2-hydroxy-propyloxy)-2-hydroxy]-phenyl}-6-[4-(2-ethyl-carboxyl)-phenylamino]-1,3,5-triazin, das 2,4-bis-{[4-(2-ethyl-hexyloxy)-2-hydroxy]-phenyl}-6-(1-methyl-pyrrol-2-yl)-1,3,5-triazin, das 2,4-Bis-{[4-tris(trimethylsiloxy-silylpropyloxy)-2-hydroxy]-phenyl}-6-(4-methoxyphenyl)-1,3,5-triazin, das 2,4-Bis-{[4-(2"-methylpropenyloxy)-2-hydroxy]-phenyl}-6-(4-methoxyphenyl)-1,3,5-triazin und das 2,4-Bis-{[4-(1',1',1',3',5',5',5'-Heptamethylsiloxy-2"-methyl-propyloxy)-2-hydroxy]-phenyl}-6-(4-methoxyphenyl)-1,3,5-triazin.

Ein vorteilhafter Breitbandfilter im Sinne der vorliegenden Erfindung ist das 2,2'-Methylen-bis-(6-(2H-benzotriazol-2-yl)-4-(1,1,3,3-tetramethylbutyl)-phenol), welches durch die chemische Strukturformel 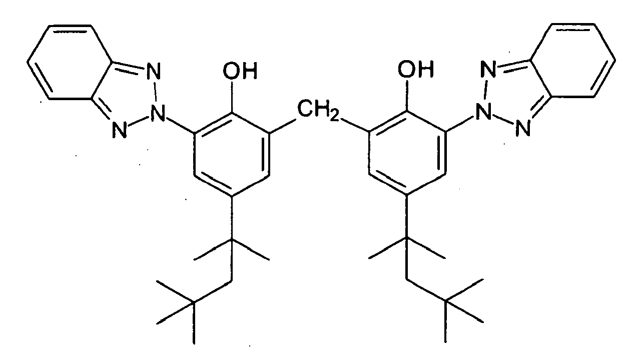 gekennzeichnet ist und unter der Handelsbezeichnung Tinosorb® M bei der CIBA-Chemikalien GmbH erhältlich ist.

Vorteilhafter Breitbandfilter im Sinne der vorliegenden Erfindung ist ferner das 2-(2H-benzotriazol-2-yl)-4-methyl-6-[2-methyl-3-[1,3,3,3-tetramethyl-1-[(trimethylsilyl)oxy]disiloxanyl]propyl]-phenol (CAS-Nr.: 155633-54-8) mit der INCI-Bezeichnung Drometrizole Trisiloxane, welches durch die chemische Strukturformel 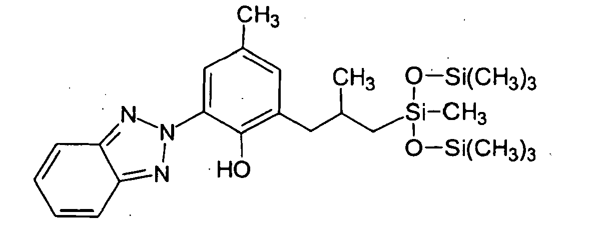 gekennzeichnet ist.

Vortailhaft ist auch

Die UV-B-Filter können öllöslich oder wasserlöslich sein. Vorteilhafte öllösliche UV-B-Filtersubstanzen sind z. B.:
3-Benzylidencampher-Derivate, vorzugsweise 3-(4-Methylbenzyliden)campher, 3-Benzylidencampher;
4-Aminobenzoesäure-Derivate, vorzugsweise 4-(Dimethylamino)-benzoesäure(2-ethylhexyl)-ester, 4-(Dimethylamino)benzoesäureamylester; 2,4,6-Trianilino-(p-carbo-2'-ethyl-1'-hexyloxy)-1,3,5-triazin;
Ester der Benzalmalonsäure, vorzugsweise 4-Methoxybenzalmalonsäuredi(2-ethylhexyl)-ester;
Ester der Zimtsäure, vorzugsweise 4-Methoxyzimtsäure(2-ethylhexyl)ester, 4-Methoxyzimtsäureisopentylester;
Derivate des Benzophenons, vorzugsweise 2-Hydroxy-4-methoxybenzophenon, 2-Hydroxy-4-methoxy-4'-methylbenzophenon, 2,2'-Dihydroxy-4-methoxybenzophenon sowie an Polymere gebundene UV-Filter.

Vorteilhafte wasserlösliche UV-B-Filtersubstanzen sind z. B.:
Salze der 2-Phenylbenzimidazol-5-sulfonsäure, wie ihr Natrium-, Kalium- oder ihr Triethanolammonium-Salz, sowie die Sulfonsäure selbst;
Sulfonsäure-Derivate des 3-Benzylidencamphers, wie z. B. 4-(2-Oxo-3-bornylidenmethyl)-benzolsulfonsäure, 2-Methyl-5-(2-oxo-3-bornylidenmethyl)sulfonsäure und deren Salze.

Eine weiterere erfindungsgemäß vorteilhaft zu verwendende Lichtschutzfiltersubstanz ist das Ethylhexyl-2-cyano-3,3-diphenylacrylat (Octocrylen), welches von BASF unter der Bezeichnung Uvinul® N 539 erhältlich ist und sich durch folgende Struktur auszeichnet:

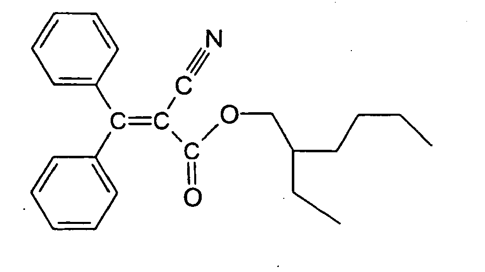

Es kann auch von erheblichem Vorteil sein, polymergebundene oder polymere UV-Filtersubstanzen in Zubereitungen gemäß der vorliegenden Erfindung zu verwenden, insbesondere solche, wie sie in der WO-A-92/20690 beschrieben werden.

Ferner kann es gegebenenfalls von Vorteil sein, erfindungsgemäß weitere UV-A- und/oder UV-B-Filter in kosmetische oder dermatologische Zubereitungen einzuarbeiten, beispielsweise bestimmte Salicylsäurederivate wie 4-Isopropylbenzylsalicylat, 2-Ethylhexylsalicylat (= Octylsalicylat), Homomenthylsalicylat.

Die Liste der genannten UV-Filter, die im Sinne der vorliegenden Erfindung eingesetzt werden können, soll selbstverständlich nicht limitierend sein.

Erfindungsgemäße kosmetische und dermatologische Zubereitungen enthalten außerdem vorteilhaft, wenngleich nicht zwingend, anorganische Pigmente auf Basis von Metalloxiden und/oder anderen in Wasser schwerlöslichen oder unlöslichen Metallverbindungen, insbesondere der Oxide des Titans (TiO₂), Zinks (ZnO), Eisens (z.B. Fe₂O₃), Zirkoniums (ZrO₂), Siliciums (SiO₂), Mangans (z.B. MnO), Aluminiums (Al₂O₃), Cers (z.B. Ce₂O₃), Mischoxiden der entsprechenden Metalle sowie Abmischungen aus solchen Oxiden. Besonders bevorzugt handelt es sich um Pigmente auf der Basis von TiO₂.

Die anorganischen Pigmente liegen erfindungsgemäß in hydrophober Form vor, d.h., dass sie oberflächlich wasserabweisend behandelt sind. Diese Oberflächenbehandlung kann darin bestehen, dass die Pigmente nach an sich bekannten Verfahren mit einer dünnen hydrophoben Schicht versehen werden.

Eines solcher Verfahren besteht beispielsweise darin, dass die hydrophobe Oberflächenschicht nach einer Reaktion gemäß

n TiO₂ + m (RO)₃Si-R'-> n TiO₂ (oberfl.)

erzeugt wird. n und m sind dabei nach Belieben einzusetzende stöchiometrische Parameter, R und R' die gewünschten organischen Reste. Beispielsweise in Analogie zu DE-OS 33 14 742 dargestellte hydrophobisierte Pigmente sind von Vorteil.

Vorteilhafte TiO₂-Pigmente sind beispielsweise unter den Handelsbezeichnungen T 805 von der Firma Degussa erhältlich.

Die Gesamtmenge an anorganischen Pigmenten, insbesondere hydrophoben anorganischen Mikropigmenten in den fertigen kosmetischen oder dermatologischen Zubereitungen wird vorteilhaft aus dem Bereich von 0,1 - 30 Gew.-%, bevorzugt 0,1 - 10,0, insbesondere 0,5 - 6,0 Gew.-% gewählt, bezogen auf das Gesamtgewicht der Zubereitungen.

Erfindungsgemäß können die kosmetischen und/oder dermatologischen Lichtschutzformulierungen wie üblich zusammengesetzt sein und dem kosmetischen und/oder dermatologischen Lichtschutz, ferner zur Behandlung, der Pflege und der Reinigung der Haut und/oder der Haare und als Schminkprodukt in der dekorativen Kosmetik dienen.

Zur Anwendung werden die kosmetischen und dermatologischen Zubereitungen erfindungsgemäß in der für Kosmetika üblichen Weise auf die Haut und/oder die Haare in ausreichender Menge aufgebracht.

Besonders bevorzugt sind solche kosmetischen und dermatologischen Zubereitungen, die in der Form eines Sonnenschutzmittels vorliegen. Vorteilhaft können diese zusätzlich mindestens einen weiteren UVA-Filter und/oder mindestens einen weiteren UVB-Filter und/oder mindestens ein anorganisches Pigment, bevorzugt ein anorganisches Mikropigment, enthalten.

Die kosmetischen und dermatologischen Zubereitungen können erfindungsgemäß kosmetische Hilfsstoffe enthalten, wie sie üblicherweise in solchen Zubereitungen verwendet werden, z.B. Konservierungsmittel, Bakterizide, Parfüme, Substanzen zum Verhindern des Schäumens, Farbstoffe, Pigmente, die eine färbende Wirkung haben, Verdickungsmittel, anfeuchtende und/oder feuchhaltende Substanzen, Fette, Öle, Wachse oder andere übliche Bestandteile einer kosmetischen oder dermatologischen Formulierung wie Alkohole, Polyole, Polymere, Schaumstabilisatoren, Elektrolyte, organische Lösungsmittel oder Silikonderivate.

Ein zusätzlicher Gehalt an üblichen Antioxidantien ist im allgemeinen bevorzugt. Erfindungsgemäß können als günstige Antioxidantien alle für kosmetische und/oder dermatologische Anwendungen geeigneten oder gebräuchlichen Antioxidantien verwendet werden.

Die Menge der vorgenannten Antioxidantien (eine oder mehrere Verbindungen) in den Zubereitungen beträgt vorzugsweise 0,001 bis 30 Gew.-%, besonders bevorzugt 0,05 - 20 Gew.-%, insbesondere 1 - 10 Gew.-%, bezogen auf das Gesamtgewicht der Zubereitung.

Sofern die kosmetische oder dermatologische Zubereitung im Sinne der vorliegenden Erfindung eine Lösung oder Emulsion oder Dispersion darstellt, können als Lösungsmittel verwendet werden:
- Wasser oder wäßrige Lösungen
- Öle, wie Triglyceride der Caprin- oder der Caprylsäure, vorzugsweise aber Rizinusöl;
- Fette, Wachse und andere natürliche und synthetische Fettkörper, vorzugsweise Ester von Fettsäuren mit Alkoholen niedriger C-Zahl, z.B. mit Isopropanol, Propylenglykol oder Glycerin, oder Ester von Fettalkoholen mit Alkansäuren niedriger C-Zahl oder mit Fettsäuren;
- Alkohole, Diole oder Polyole niedriger C-Zahl, sowie deren Ether, vorzugsweise Ethanol, Isopropanol, Propylenglykol, Glycerin, Ethylenglykol, Ethylenglykolmonoethyl- oder -monobutylether, Propylenglykolmonomethyl, -monoethyl- oder - monobutylether, Diethylenglykolmonomethyl- oder -monoethylether und analoge Produkte.

Insbesondere werden Gemische der vorstehend genannten Lösungsmittel verwendet. Bei alkoholischen Lösungsmitteln kann Wasser ein weiterer Bestandteil sein.

Sofern die kosmetische oder dermatologische Zubereitung im Sinne der vorliegenden Erfindung eine Lösung oder Emulsion oder Dispersion darstellt, können als Lösungsmittel verwendet werden:
- Wasser oder wäßrige Lösungen
- Öle, wie Triglyceride der Caprin- oder der Caprylsäure, vorzugsweise aber Rizinusöl;
- Fette, Wachse und andere natürliche und synthetische Fettkörper, vorzugsweise Ester von Fettsäuren mit Alkoholen niedriger C-Zahl, z.B. mit Isopropanol, Propylenglykol oder Glycerin, oder Ester von Fettalkoholen mit Alkansäuren niedriger C-Zahl oder mit Fettsäuren;
- Alkohole, Diole oder Polyole niedriger C-Zahl, sowie deren Ether, vorzugsweise Ethanol, Isopropanol, Propylenglykol, Glycerin, Ethylenglykol, Ethylenglykolmonoethyl- oder -monobutylether, Propylenglykolmonomethyl, -monoethyl- oder -monobutylether, Diethylenglykolmonomethyl- oder -monoethylether und analoge Produkte.

Insbesondere werden Gemische der vorstehend genannten Lösungsmittel verwendet. Bei alkoholischen Lösungsmitteln kann Wasser ein weiterer Bestandteil sein.

Als Treibmittel für aus Aerosolbehältern versprühbare kosmetische und/oder dermatologische Zubereitungen im Sinne der vorliegenden Erfindung sind die üblichen bekannten leichtflüchtigen, verflüssigten Treibmittel, beispielsweise Kohlenwasserstoffe (Propan, Butan, Isobutan) geeignet, die allein oder in Mischung miteinander eingesetzt werden können. Auch Druckluft ist vorteilhaft zu verwenden.

Natürlich weiß der Fachmann, dass es an sich nichttoxische Treibgase gibt, die grundsätzlich für die Verwirklichung der vorliegenden Erfindung in Form von Aerosolpräparaten geeignet wären, auf die aber dennoch wegen bedenklicher Wirkung auf die Umwelt oder sonstiger Begleitumstände verzichtet werden sollte, insbesondere Fluorkohlenwasserstoffe und Fluorchlorkohlenwasserstoffe (FCKW).

Kosmetische Zubereitungen im Sinne der vorliegenden Erfindung können auch als Gele vorliegen, die neben einem wirksamen Gehalt am erfindungsgemäßen Wirkstoff und dafür üblicherweise verwendeten Lösungsmitteln, bevorzugt Wasser, noch organische Verdickungsmittel, z.B. Gummiarabikum, Xanthangummi, Natriumalginat, Cellulose-Derivate, vorzugsweise Methylcellulose, Hydroxymethylcellulose, Hydroxyethylcellulose, Hydroxypropylcellulose, Hydroxypropylmethylcellulose oder anorganische Verdickungsmittel, z. B. Aluminiumsilikate wie beispielsweise Bentonite, oder ein Gemisch aus Polyethylenglykol und Polyethylenglykoistearat oder -distearat, enthalten. Das Verdickungsmittel ist in dem Gel z.B. in einer Menge zwischen 0,1 und 30 Gew.-%, bevorzugt zwischen 0,5 und 15 Gew.-%, enthalten.

Es ist erfindungsgemäß vorteilhaft, außer den erfindungsgemäßen Kombinationen weitere öllösliche UVA-Filter und/oder UVB-Filter in der Lipidphase und/oder weitere wasserlösliche UVA-Filter und/oder UVB-Filter in der wäßrigen Phase einzusetzen.

Vorteilhaft können die Lichtschutzformulierungen erfindungsgemäß weitere Substanzen enthalten, die UV-Strahlung im UVB-Bereich absorbieren, wobei die Gesamtmenge der Filtersubstanzen z.B. 0,1 Gew.-% bis 30 Gew.-%, vorzugsweise 0,5 bis 10 Gew.-%, insbesondere 1 bis 6 Gew.-% beträgt, bezogen auf das Gesamtgewicht der Zubereitungen, um kosmetische Zubereitungen zur Verfügung zu stellen, die die Haut vor dem gesamten Bereich der ultravioletten Strahlung schützen. Sie können auch als Sonnenschutzmittel dienen.

Die nachfolgenden Beispiele sollen die vorliegende Erfindung verdeutlichen, ohne sie einzuschränken. Alle Mengenangaben, Anteile und Prozentanteile sind, soweit nicht anders angegeben, auf das Gewicht und die Gesamtmenge bzw. auf das Gesamtgewicht der Zubereitungen bezogen.

### Beispiel 1

### Kühlende Tagescreme mit Lichtschutz

| Fettphase: | |
|---|---|
| Glycerylstearat | 1,3% |
| Stearinsäure | 2,5% |
| Mikrokristallines Wachs | 2% |
| Cetylalkohol | 1% |
| Ethylhexylmethoxycinnamat (Parsol MCX) | 5% |
| Cyclomethicon | 1% |
| Ethylhexyl Triazon (Uvinol T 150) | 2% |
| Stearylalkohol | 2% |
| Bis-Ethylhexyloxyphenol Methoxyphenyl Triazin (Tinosorb S) | 1% |
| Tocopherylacetat | 0,5% |
| 2-Isopropyl-5-Methyl-Cyclohexancarbonyl-D-Alaninmethylester | 0,5% |

| Wasserphase: | |
|---|---|
| Glycerin | 7% |
| EDTA | 0.2% |
| Butylenglykol | 3% |
| Panthenol | 1% |
| Phenoxyethanol | 0,4% |
| Methylparaben | 0,2% |
| dem. Wasser | ad 100% |

| Verdickerphase: | |
|---|---|
| Carbomer | 0,15% |
| Dimethicon | 2% |
| | |
| Natriumstärkeoctenylsuccinat (Füllstoff) | 2% |
| Parfum | 0,3% |
| NaOH | q.s. |

### Herstellung:

Verdickerphase, Parfum, Füllstoffe, sowie Fett- und Wasserphase werden getrennt eingewogen. Fett- und Wasserphase werden auf ca. 80°C erhitzt. Bei ca. 78°C werden die Phasen vereinigt, die Verdickerphase wird zugefügt und ggf. wird NaOH zum Neutralisieren zugefügt. Nach dem Abkühlen auf 65°C wird homogenisiert. Anschließend wird die entstandene Emulsion kaltgerührt bis sie auf RT abgekühlt ist. Währenddessen wird bei ca. 45°C die Füllstoffphase und bei ca. 30°C das Parfum zugegeben und der pH Wert nach Bedarf eingestellt.

### Beispiel 2

### Kühlende Lichtschutzcreme

| | |
|---|---|
| Caprylsäure/Caprinsäuretriglyceride | 1% |
| Cetylalkohol | 3% |
| Ethylhexylmethoxycinnamat (Parsol MCX) | 5% |
| Hydrierte Kokosfettsäureglyceride | 2% |
| Ethylhexyl Triazon (Uvinol T 150) | 1,5% |
| Stearaylalkohol | 3% |
| Glkycerylstearatcitrat | 2% |
| Dicaprylylether | 4% |
| Bis-Ethylhexyloxyphenol Methoxyphenyl Triazin (Tinosorb S) | 2% |
| BHT | 0,03% |
| Glycerin | 7% |
| EDTA | 0,2% |
| Butylenglykol | 3% |
| Panthenol | 1% |
| Phenoxyethanol | 0,4% |
| Methylparaben | 0,2% |
| Carbomer | 0,15% |
| Dimethicon | 2% |
| Natriumstärkeoctenylsuccinat (Füllstoff) | 2% |
| Parfum | q.s. |
| NaOH | q.s. |
| 2-Isopropyl-5-Methyl-Cyclohexancarbonyl-D-Alaninmethylester | 0,25% |
| Wasser | ad 100 |

### Beispiel 3

### Kühlende Emulsion mit Lichtschutz und hautpflegenden Eigenschaften

| | |
|---|---|
| Glycerylstearat | 2,6% |
| Caprylsäure/Caprinsäuretriglyceride | 2 % |
| Glycerin | 5 % |
| Tocopherylacetat | 0,5 % |
| Cetearylalcohol | 2 % |
| PEG-40 Stearat | 0,8% |
| Trisodium EDTA | 1 % |
| Ethylhexylmethoxycinnamat | 5% |
| Dimethicon | 1 % |
| Cyclomethicon | 2 % |
| Myristylmyristat | 0,5 % |
| Butylmethoxydibenzoylmethan | 2 % |
| Phenylbenzimidazolsulfonsäure | 2 % |
| Carbomer | 0,1 % |
| Methylpalmitat | 1% |
| 2-Isopropyl-5-Methyl-Cyclohexancarbonyl-D-Alaninmethylester | 0.75% |
| Wasser | ad 100 % |

### Beispiel 4

### Tagescrème

| | |
|---|---|
| Glycerylstearat | 2,5% |
| Glycerin | 5% |
| Tocopherylacetat | 0,5% |
| Cetearylalcohol | 2% |
| PEG-40 Stearat | 0,75% |
| Ethylhexylmethoxycinnamat | 5% |
| Cyclomethicon | 5% |
| Benzophenon-3 | 3% |
| MyristylmMyristat | 1 % |
| C₁₂₋₁₅ Alkylbenzoat | 2% |
| Phenylbenzimidazol Sulfonic Acid | 2% |
| Carbomer | 0,15% |
| Triisostearin | 2% |
| Methylpalmitat | 2% |
| Diazolidinylharnstoff | q.s. |
| 2-Isopropyl-5-Methyl-Cyclohexancarbonyl-D-Alaninmethylester | 0,75% |
| Aqua | ad 100 % |

### Beispiel 5

### Kühlende Körperlotion

| | |
|---|---|
| Cetylalkohol | 3% |
| Glycerylstearat SE | 1,5% |
| Butylmethoxydibenzoylmethan | 3 % |
| Hydrierte Kokosfettglyceride (Hydrogenated Coco Glycerides) | 1 % |
| Ethylhexylsalicylat | 4 % |
| Ethylhexylmethoxycinnamat | 7 % |
| BHT | 0,05% |
| EDTA | 0,2 % |
| Tocopherylacetat | 0,5 % |
| Benzophenon-3 | 3 % |
| Phenoxyethanol | 0,4 % |
| Dimethicon | 2 % |
| Nylon 12 | 2 % |
| Distärkephosphat | 3 % |
| Xanthangummi | 0,4 % |
| Glycerin | 7 % |
| Phenylbenimidazolsulfonsäure | 2 % |
| Ethanol | 4 % |
| Ethylhexyglycerin | 0,5 % |
| Ethylparaben | 0,1 |
| Methylparaben | 0,2 |
| Propylparaben | 0,1 |
| 2-Isopropyl-5-Methyl-Cyclohexancarbonyl-D-Alaninmethylester | 0,5 |
| Aqua | ad 100 |

## Patentansprüche

1. Kosmetische oder dermatologische Zubereitungen mit einer Wirkstoffkombination aus 2-Isopropyl-5-Methyl-Cyclohexancarbonyl-D-Alaninmethylester und einer oder mehreren Lichtschutzfiltersubstanzen.

2. Zubereitungen nach Anspruch 1, **dadurch gekennzeichnet, dass** die Lichtschutzfiltersubstanz oder Lichtschutzfiltersubstanzen gewählt wird oder werden aus der Gruppe der Derivate des Benzylidencamphers.

3. Zubereitungen nach Anspruch 1, **dadurch gekennzeichnet, dass** die Lichtschutzfiltersubstanz oder Lichtschutzfiltersubstanzen gewählt wird oder werden aus der Gruppe der Benzophenone.

4. Zubereitungen nach Anspruch 1, **dadurch gekennzeichnet, dass** die Lichtschutzfiltersubstanz oder Lichtschutzfiltersubstanzen gewählt wird oder werden aus der Gruppe der Derivate der Zimtsäure.

5. Zubereitungen nach Anspruch 1, **dadurch gekennzeichnet, dass** die Lichtschutzfiltersubstanz oder Lichtschutzfiltersubstanzen gewählt wird oder werden aus der Gruppe der Derivate der Salicylsäure.

6. Zubereitungen nach Anspruch 1, **dadurch gekennzeichnet, dass** die Lichtschutzfiltersubstanz oder Lichtschutzfiltersubstanzen gewählt wird oder werden aus der Gruppe der Derivate der Triazone und Triazine.

7. Zubereitungen nach Anspruch 1, **dadurch gekennzeichnet, dass** die Lichtschutzfiltersubstanz oder Lichtschutzfiltersubstanzen gewählt wird oder werden aus der Gruppe Butylmethoxydibenzoylmethan, der Bis-Imidazylate und Octocrylen

8. Zubereitungen nach einem der vorstehenden Ansprüche, **dadurch gekennzeichnet, dass** sie 0,001 Gew.-% bis 10 Gew.-%. bevorzugt 0,01 Gew.-% bis 1,0 Gew.-%, insbesondere 0,05 - 0,5 Gew.-%, bezogen auf das Gesamtgewicht der Zubereitungen, an 2-isopropyl-5-Methyl-Cyclohexancarbonyl-D-Alaninmethylester enthalten.

9. Zubereitungen nach einem der vorstehenden Ansprüche, **dadurch gekennzeichnet, dass** sie 0,001 Gew.-% bis 25 Gew.-%, bevorzugt 0,05 Gew.-% bis 15 Gew.-%, insbesondere 0,1 - 10,0 Gew.-%, bezogen auf das Gesamtgewicht der Zubereitungen, an einer oder mehreren Lichtschutzfiltersubstanzen enthalten.

## Claims

1. Cosmetic or dermatological preparations with an active ingredient combination of 2-isopropyl-5-methylcyclohexanecarbonyl-D-alanine methyl ester and one or more light protective filter substances.

2. Preparations according to Claim 1, **characterized in that** the light protective filter substance or light protective filter substances is or are selected from the group of derivatives of benzylidenecamphor.

3. Preparations according to Claim, 1, **characterized in that** the light protective filter substance or light protective filter substances is or are selected from the group of benzophenones.

4. Preparations according to Claim 1, **characterized in that** the light protective filter substance or light protective filter substances is or are selected from the group of derivatives of cinnamic acid.

5. Preparations according to Claim 1, **characterized in that** the light protective filter substance or light protective filter substances is or are selected from the group of derivatives of salicylic acid.

6. Preparations according to Claim 1, **characterized in that** the light protective filter substance or light protective filter substances is or are selected from the group of derivatives of triazones and triazines.

7. Preparations according to Claim 1, **characterized in that** the light protective filter substance or light protective filter substances is or are selected from the group consisting of butylmethoxydibenzoylmethane, the bis-imidazylates and octocrylene.

8. Preparations according to one of the preceding claims, **characterized in that** they comprise 0.001% by weight to 10% by weight, preferably 0.01% by weight to 1.0% by weight, in particular 0.05-0.5% by weight, based on the total weight of the preparations, of 2-isopropyl-5-methylcyclohexanecarbonyl-D-alanine methyl ester.

9. Preparations according to one of the preceding claims, **characterized in that** they comprise 0.001% by weight to 25% by weight, preferably 0.05% by weight to 15% by weight, in particular 0.1-10.0% by weight, based on the total weight of the preparations, of one or more light protective filter substances.

## Revendications

1. Préparations cosmétiques ou dermatologiques avec une combinaison de substances actives d'ester méthylique de la 2-isopropyl-5-méthylcyclohexanecarbonyl-D-alanine et d'une ou de plusieurs substances filtre de protection contre la lumière.

2. Préparations selon la revendication 1, **caractérisées en ce que** la ou les substance(s) filtre de protection contre la lumière est/sont choisie(s) dans le groupe des dérivés du benzylidènecamphre.

3. Préparations selon la revendication 1, **caractérisées en ce que** la ou les substance(s) filtre de protection contre la lumière est/sont choisie(s) dans le groupe des benzophénones.

4. Préparations selon la revendication 1, **caractérisées en ce que** la ou les substance(s) filtre de protection contre la lumière est/sont choisie(s) dans le groupe des dérivés de l'acide cinnamique.

5. Préparations selon la revendication 1, **caractérisées en ce que** la ou les substance(s) filtre de protection contre la lumière est/sont choisie(s) dans le groupe des dérivés de l'acide salicylique.

6. Préparations selon la revendication 1, **caractérisées en ce que** la ou les substance(s) filtre de protection contre la lumière est/sont choisie(s) dans le groupe des dérivés des triazones et des triazines.

7. Préparations selon la revendication 1, **caractérisées en ce que** la ou les substance(s) filtre de protection contre la lumière est/sont choisie(s) dans le groupe formé par le butylméthoxydibenzoylméthane, les bis-imidazylates et l'octocrylène.

8. Préparations selon l'une quelconque des revendications précédentes, **caractérisées en ce qu'**elles contiennent 0,001% en poids à 10% en poids, de préférence 0,01% en poids à 1,0% en poids, en particulier 0,05-0,5% en poids, par rapport au poids total des préparations, d'ester méthylique de la 2-isopropyl-5-méthyl-cyclohexanecarbonyl-D-alanine.

9. Préparations selon l'une quelconque des revendications précédentes, **caractérisées en ce qu'**elles contiennent 0,001% en poids à 25% en poids, de préférence 0,05% en poids à 15% en poids, en particulier 0,1-10,0% en poids, par rapport au poids total des préparations, d'une ou de plusieurs substance(s) filtre de protection contre la lumière.
